Europäisches Patentamt

European Patent Office (11) Numéro de publication: **0 265 352**
**B1**

Office européen des brevets

(12) **FASCICULE DE BREVET EUROPEEN**

(45) Date de publication du fascicule du brevet: **30.01.91** (51) Int. Cl.⁵: **A 61 M 1/16**

(21) Numéro de dépôt: **87420272.4**

(22) Date de dépôt: **07.10.87**

(54) **Procédé de pompage pour rein artificiel de deux liquides en quantités égales et dispositif de pompage en faisant application.**

(30) Priorité: **20.10.86 FR 8614849**

(43) Date de publication de la demande:
**27.04.88 Bulletin 88/17**

(45) Mention de la délivrance du brevet:
**30.01.91 Bulletin 91/05**

(84) Etats contractants désignés:
**BE DE ES FR GB IT NL SE**

(56) Documents cités:
**FR-A-2 368 283**

(73) Titulaire: **HOSPAL INDUSTRIE**
**7, Avenue Lionel Terray**
**F-69330 Meyzieu (FR)**

(72) Inventeur: **Cortial, Jean Loup**
**45, rue de la Balme**
**F-69003 Lyon (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**Description**

La présente invention concerne le domaine technique du rein artificiel. Plus particulièrement, la présente invention concerne un procédé pour le pompage en quantités égales et avec un débit continu de deux liquides dans deux conduites séparées et son application à un rein artificiel.

La présente invention concerne également un dispositif permettant la mise en oeuvre d'un tel procédé dans un rein artificiel.

On connait déjà, dans l'état de la technique des reins artificiels comprenant des dispositifs de pompage de deux liquides en quantités égales.

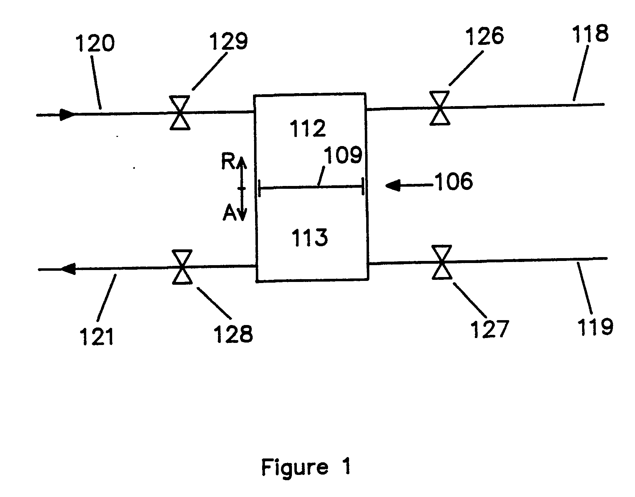

Il s'agit, par exemple, d'un dispositif de pompage à deux chambres complémentaires associé à quatre vannes, tel que représenté à la figure 1. Le fonctionnement d'un tel dispositif se fait en deux temps. Pendant un premier temps, les vannes 126 et 127 sont fermées alors que les vannes 128 et 129 sont ouvertes. Le piston 109 séparant les deux chambres complémentaires 112 et 113, est déplacé dans le sens A, de façon à aspirer un premier liquide dans la chambre 112, ce qui provoque, dans le même temps, le refoulement à partir de la chambre 113 d'une égale quantité d'un second liquide. Dans un second temps, les vannes 128 et 129 sont fermées alors que les vannes 126 et 127 sont ouvertes.

Le piston 109 est alors déplacé dans le sens R de façon à refouler à partir de la chambre 112 dans la canalisation 118 und quantité du premier liquide égale à la quantité du second liquide aspiré simultanément dans la chambre 113 et provenant de la canalisation 119.

Avec un tel dispositif, il est possible de pomper dans deux conduites séparées deux liquides en quantités égales, mais les débits dans les quatre canalisations 118, 119, 120, 121 sont discontinus.

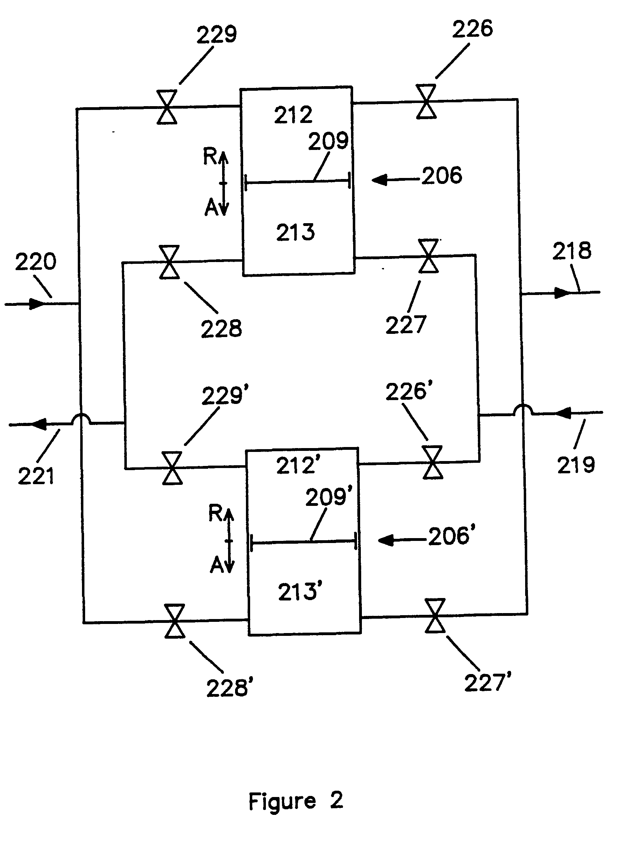

Pour pallier à cet inconvénient, la solution proposée dans l'art antérieur est de doubler le dispositif; on obtient alors un système constitué de deux dispositifs de pompage à deux chambres complémentaires associés à huit vannes, tel que représenté à la figure 2.

Le fonctionnement d'un tel dispositif est le suivant:

Dans un premier temps, les vannes 226, 227, 228' et 229' sont fermées alors que les vannes 228, 229, 226' et 227' sont ouvertes. Les pistons 209 et 209' sont alors déplacés dans le sens A. Le déplacement du piston 209 entraine l'aspiration dans la chambre 212 du premier liquide à partir de la canalisation 220 d'entrée du premier liquide ainsi que le refoulement du second liquide à partir de la chambre 213 dans la canalisation 221.

Dans le même temps, le mouvement du piston 209' entraine le refoulement du premier liquide à partir de la chambre 213' vers la canalisation 218, ainsi que l'aspiration dans la chambre 212' du second liquide provenant de la canalisation 219. Dans un second temps, les vannes 226, 227, 228' et 229' sont ouvertes et les vannes 228, 229, 226' et 227' sont fermées.

Les mouvements des pistons 209 et 209' sont cette fois inversés. Le mouvement du piston 209' entraine une aspiration du premier liquide dans la chambre 213' et un refoulement du second liquide de la chambre 212' vers la canalisation 221. Simultanément, le mouvement du piston 209 entraine l'aspiration dans la chambre 213 du second liquide provenant de la canalisation 219 en même temps que le refoulement à partir de la chambre 212 du premier liquide dans la canalisation 218.

Un système analogue à celui qui vient d'être décrit fait l'objet de la demande de bevet allemande DE—A—2 634 238, avec cependant la différence qu'il ne s'agit pas d'un dispositif de pompage à pistons mais d'un dispositif de substitution de volumes identiques entre un premier liquide qui est le liquide de dialyse frais et un second liquide qui est le liquide de dialyse usagé. Les chambres des dispositifs de substitution ne sont pas séparées par des pistons mais par des membranes élastiques. La circulation du liquide de dialyse est dûe, non pas au déplacement des pistons, mais à l'action de deux pompes (1, 2) qui poussent le liquide de dialyse.

Grâce à de tels systèmes, on obtient des débits continus dans les canalisations 218, 219, 220, 221.

Ces systèmes permettent, en principe, de pomper en quantités égales et avec un débit continu, deux liquides dans deux conduites séparées. Cependant, à chaque ouverture ou fermeture de vannes (226, 227, 228, 229, 226', 227', 228', 229'), il peut se produire une petite erreur. Lorsque la fréquence d'ouverture et de fermeture des vannes est importante, l'accumulation de ces petites erreurs peut conduire à une erreur totale importante.

Le brevet FR—A—2 368 283 décrit également un dispositif de pompage du liquide de dialyse frais et du liquide de dialyse usagé en quantités égales. Cependant, ce système présenté également l'inconvénient de nécessiter 2 jeux de 4 soupapes (les soupapes 64, 74, 84, 94 et les soupapes 65, 75, 85, 95) dont les cycles d'ouverture et de fermeture alternent avec la possibilité à chaque changement d'état des soupapes de commetre une erreur sur la quantité de liquide pompée.

C'est justement l'objectif de la présente invention que de pallier aux inconvénients de l'art antérieur.

Un objet de la présente invention est de proposer un procédé et un dispositif de pompage de quantités égales de deux liquides dans deux conduits séparées.

Un autre objet de la présente invention est de proposer un procédé et un dispositif de pompage de deux liquides permettant de maintenir un débit continu dans les canalisations d'aspiration et de refoulement des deux liquides.

D'autres objets et avantages de la présente invention apparaitront au cours de la description qui va suivre.

Il a maintenant été trouvé, et c'est ce qui fait l'objet de la présente invention:

un procédé de pompage pour rein artificiel,

d'un premier et d'un second liquide en quantités égales et avec un débit continu selon lequel on aspire et on refoule simultanément, en quantités égales, un premier et un second liquide grâce à un dispositif de pompage à 2 chambres principales complémentaires, la première chambre étant destinée au premier liquide et communiquant avec une canalisation d'aspiration par un premier dispositif d'obturation et avec une canalisation de refoulement par un second dispositif d'obturation, la seconde chambre étant destinés au second liquide est communiquant avec une canalisation d'aspiration par un troisième dispositif d'obturation et avec une canalisation de refoulement par un quatrième dispositif d'obturation caractérisé en ce que:

—on régule le débit du premier liquide grâce à une capacité de régulation comprenant deux chambres auxiliaires dont les volumes varient simultanément et en sens opposé du volume de la chambre principale, la quantité de liquide déplacé lors de la variation de volume de la chambre principale étant double de la quantité de liquide déplacé lors de la variation de volume de chacune des chambres auxiliaires correspondantes, l'une des chambres auxiliaires étant reliée à la canalisation d'aspiration du premier liquide alors que l'autre chambre auxiliaire est reliée à la canalisation de refoulement dudit premier liquide

—on régule le débit du second liquide grâce à une capacité de régulation comprenant deux chambres auxiliaires dont les volumes varient simultanément et en sens opposé du volume de la chambre principale correspondante, la quantité de liquide déplacé lors de la variation de volume de la chambre principale étant double de la quantité de liquide déplace lors de la variation de volume de chacune des chambres auxiliaires correspondantes, l'une des chambres auxiliaires étant reliée à la canalisation d'aspiration du second liquide alors que l'autre chambre auxiliaire est reliée à la canalisation de refoulement dudit second liquide.

L'invention vise également un dispositif de pompage pour rein artificiel d'un premier et d'un second liquide en quantités égales et avec un débit sensiblement constant comprenant un dispositif principal de pompage à deux chambres principales complémentaires, la chambre destinée au premier liquide communiquant par un dispositif d'obturation avec une canalisation d'aspiration du premier liquide et par un dispositif d'obturation avec une canalisation de refoulement du premier liquide, la chambre destinée au second liquide communiquant par un dispositif d'obturation avec une canalisation d'aspiration du second liquide et par un dispositif d'obturation avec une canalisation de refoulement du second liquide, caractérisé en ce qu'il comporte en outre:

—une capacité de régulation du premier liquide constituée par deux chambres auxiliaires dont les volumes varient simultanément et en sens opposé du volume de la chambre principale correspondante, la quantité de liquide déplacé lors de la variation de volume de la chambre principale étant double de la quantité de liquide déplacé lors de la variation de volume de chacune des chambres auxiliaires l'une des chambres auxiliaires étant reliée à la canalisation d'aspiration du premier liquide en amont d'un premier dispositif d'obturation alors que l'autre chambre auxiliaire est reliée à la canalisation de refoulement du premier liquide en aval d'un second dispositif d'obturation.

—une capacité de régulation du second liquide constituée par deux chambres auxiliaires dont les volumes varient simultanément et en sens opposé du volume de la chambre principale correspondante, la quantité de liquide déplacé lors se la variation de volume de la chambre principale étant double de la quantité de liquide déplacé lors de la variation de volume de chacune des chambres auxiliaires, l'une des chambres auxiliaires étant reliée à la canalisation d'aspiration du second liquide en amont d'un troisième dispositif d'obturation alors que l'autre chambre auxiliaire est reliée à la canalisation de refoulement du seconde liquide en aval d'un quatrième dispositif d'obturation.

—des moyens d'entrainement du dispositif principal de pompage.

La présente invention sera mieux comprise à la lecture de la description qui va suivre en référence aux dessins annexés qui illustrent, de façon schématique et sans échelle déterminée, des exemples de réalisation de la présente invention.

La figure 1 représente, de façon schématique, un dispositif connu de pompage à deux chambres complémentaires associé à quatre vannes.

La figure 2 représente, de façon schématique, une association connue de deux dispositifs de pompage selon la figure 1.

La figure 3 représente, de façon schématique, un premier mode de réalisation de la présente invention appliquée au pompage du liquide de dialyse dans un rein artificiel.

La figure 4 représente les courbes de variation de débit en fonction du temps, dans les canalisations du premier liquide.

La figure 5 représente les courbes de variation du débit en fonction du temps, dans les canalisations du second liquide.

La figure 6 représente, de façon schématique, un second mode de réalisation de la présente invention.

La figure 7 représente, de façon schématique, un troisième mode de réalisation de la présente invention.

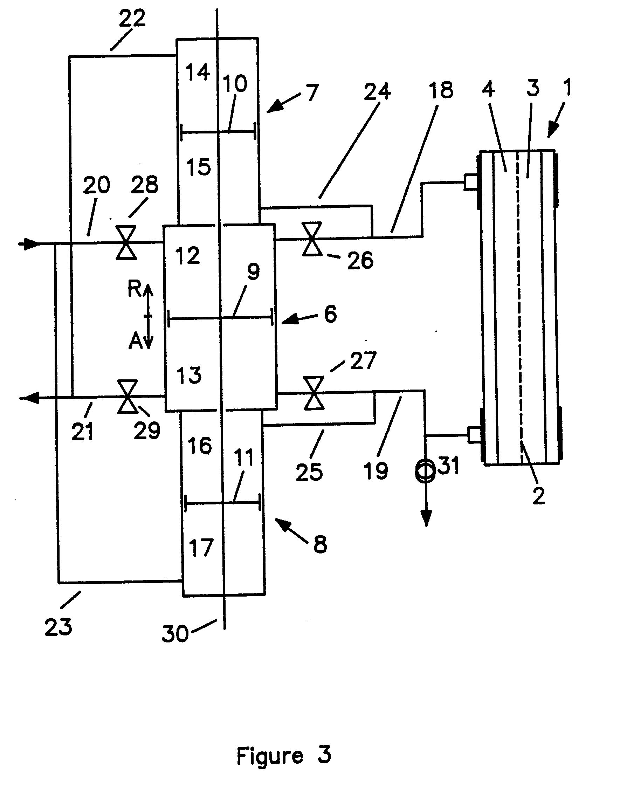

En se référant à la figure 3, on voit que le dispositif selon la présente invention peut être utilisé dans un rein artificiel comportant un hémodialyseur (1) séparé en deux compartiments (3, 4) par une membrane semi-perméable (2) permettant la dialyse et l'ultrafiltration du sang. Le premier compartiment (3) est destiné à la circulation extracorporelle du sang, alors que le second compartiment (4) est destiné à la circulation du liquide de dialyse qui s'écoule à partir d'une source (non représentée) vers l'hémodialyseur, puis de l'hémodialyseur vers des moyens d'évacuation ou de régénération (non représentés).

On alimente le compartiment (4) de l'hémodialyseur grâce à un dispositif principal de pompage (6) qui est constitué d'un cylindre séparé en deux chambres principales complémentaires (12, 13) par un piston (9). Ce piston, qui présente de bonnes qualités d'étanchéité, est mobile à l'intérieur du cylindre. La chambre de liquide de dialyse frais 12 est reliée à une source de liquide de dialyse (non représentée) par une canalisation 20 munie d'un dispositif d'obturation 28 et à l'entrée de l'hémodialyseur par une canalisation 18 munie également d'un dispositif d'obturation 26. La chambre de liquide de dialyse usagé 13 est reliée à la sortie de l'hémodialyseur par une canalisation 19 munie d'un dispositif d'obturation 27 et à des moyens d'évacuation ou de régénération du liquide de dialyse usagé (non représentés) par une canalisation 21 munie d'un dispositif d'obturation 29.

A ce dispositif principal de pompage 6 sont associés deux cylindres auxiliaires 7, 8. Chacun des cylindres est séparé en deux chambres auxiliaires complémentaires, grâce à un piston (10, 11). Ces pistons coaxiaux, sont solidaires entre eux et solidaires du piston principal 9, grâce à une tige 30.

Dans chaque cylindre, l'une des chambres auxiliaires est destinée au liquide de dialyse frais (15, 17), alors que l'autre chambre est destinée au liquide de dialyse usagé (14, 16).

La chambre auxiliaire 14 est reliée par une canalisation 22 à la canalisation 21 d'évacuation du liquide de dialyse usagé, en aval du dispositif d'obturation 29, alors que la chambre auxiliaire 15 qui lui est complémentaire, est reliée par une canalisation 24 à la canalisation 18 d'entrée de l'hémodialyseur, en aval du dispositif d'obturation 26. La chambre auxiliaire 16 est reliée par une canalisation 25 à la canalisation 19 de sortie de l'hémodialyseur, en amont du dispositif d'obturation 27, alors que la chambre auxiliaire 17 qui lui est complémentaire, est reliée par une canalisation 23 à la canalisation 20 d'entrée du liquide de dialyse, en amont du dispositif d'obturation 28.

Les chambres auxiliaires 14 et 16 constituent une capacité de régulation du débit de liquide de dialyse usagé, alors que les chambres auxiliaires 15 et 17 constituent une capacité de régulation du débit de liquide de dialyse frais. La construction des cylindres auxiliaires est choisie de telle façon que le déplacement simultané des trois pistons entraine le déplacement dans chaque chambre principale d'une quantité de liquide double de la quantité de liquide déplacé au même moment dans chacune des chambres auxiliaires.

Dans le cas où les trois cylindres 6, 7, 8 sont des cylindres de section droite circulaire, on peut choisir les cylindres auxiliaires avec une section droite égale à la moitié de la section droite du cylindre principal.

Une fois le dispositif selon l'invention amorcé, son fonctionnement selon le mode de réalisation de la figure 3 est le suivant:

on anime la tige 30 d'un mouvement alternatif rectiligne, au moyen par exemple d'un moteur (non représenté).

Dans une première phase, que l'on appelle phase d'aspiration, les vannes 26 et 27 sont fermées alors que les vannes 28 et 29 sont ouvertes. La tige 30 est alors animée de telle façon que les pistons 9, 10 et 11 se déplacent dans le sens de la flèche A. Le déplacement du piston 9 entraine l'aspiration du liquide de dialyse frais dans la chambre 12 et le refoulement du liquide de dialyse usage à partir de la chambre 13. Le liquide de dialyse aspiré provient pour moitié de la source de liquide de dialyse (non représentée) et pour moitié de la chambre auxiliaire de régulation 17, par l'intermédiaire de la canalisation 23; le liquide mis en réserve dans cette chambre auxiliaire est en effet chassé par le déplacement du piston 11. Le liquide de dialyse usagé est refoulé pour moitié dans la chambre auxiliaire 14 par la canalisation 22, suite au déplacement du piston 10, et pour moitié dans la canalisation 21 d'évacuation. Le déplacement du piston 10 entraine simultanément le refoulement du liquide de dialyse frais à partir de la chambre auxiliaire 15 vers la canalisation 18 d'entrée de l'hémodialyseur par l'intermédiaire de la canalisation 24. Suite au déplacement du piston 11, le liquide de dialyse usagé provenant de l'hémodialyseur et circulant dans la canalisation 19 est aspiré par l'intermédiaire de la canalisation 25 dans la chambre auxiliaire 16.

Dans une seconde phase que l'on appelle phase de refoulement, la tige 30 est animée de telle façon que les pistons 9, 10 et 11 se déplacent dans le sens de la flèche R. Les vannes 26 et 27 sont ouvertes alors que les vannes 28 et 29 sont fermées.

La diminution de volume de la chambre principale 12 entraine le refoulement du liquide de dialyse frais d'une part dans la chambre auxiliaire 15 par l'intermédiaire de la canalisation 24, et d'autre part, dans la canalisation 18 d'entrée de l'hémodialyseur. L'augmentation de volume de la chambre 13 entraine l'aspiration de liquide de dialyse usagé provenant de la canalisation 19 de sortie de l'hémodialyseur, ainsi que de la chambre 16 par l'intermédiaire de la canalisation 25.

En effet, le mouvement du piston 11 chasse le liquide de dialyse usagé de la chambre auxiliaire 16 et entraine simultanément une aspiration dans la chambre auxiliaire 17 du liquide de dialyse frais provenant de la canalisation d'entrée 20 par l'intermédiaire de la canalisation 23. Dans le même temps, le mouvement du piston 10 chasse le liquide de dialyse usagé de la chambre auxiliaire 14 dans la canalisation d'évacuation 21 par l'intermédiaire de la canalisation 22.

La fermeture et l'ouverture successives des vannes (26, 27, 28, 29) ainsi que le changement de sens du déplacement de la tige 30 peuvent être commandés grâce à des contacts de fin de course solidaires de la tige et associés à un système de pilotage (non représenté) de tout type connu en soi, par exemple au moyen d'un microprocesseur.

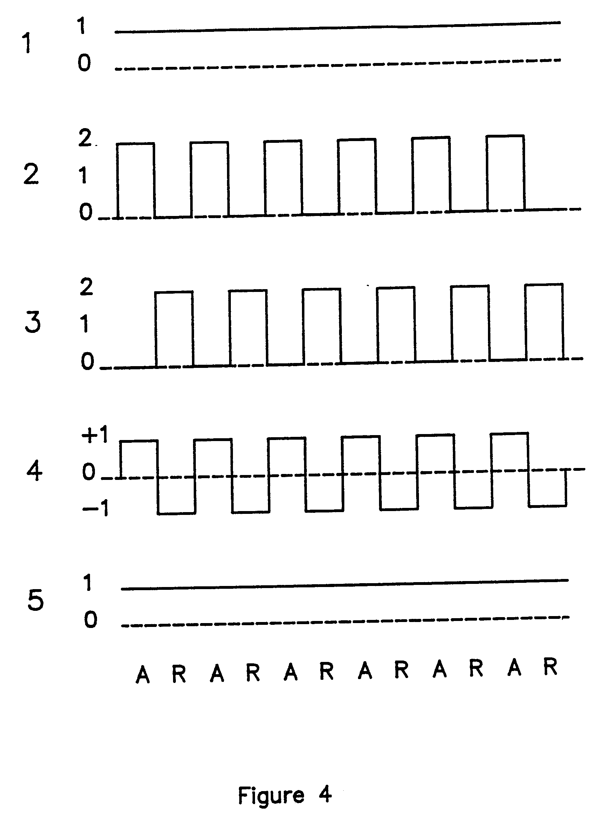

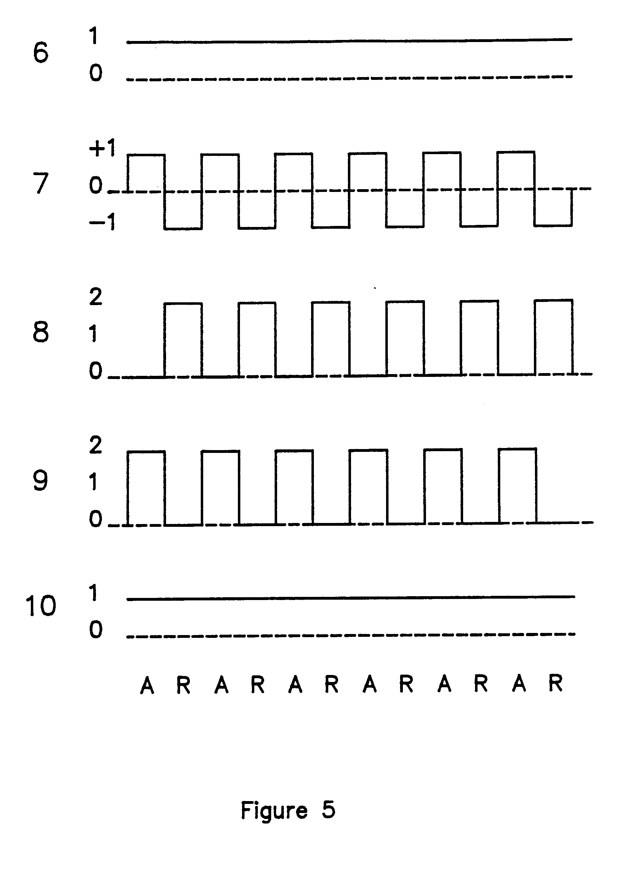

Si l'on se réfère aux courbes des figures 4 et 5 pour l'observation des débits dans les différentes canalisations, on remarque d'une part que la quantité de liquide de dialyse frais (ou premier liquide) aspiré est égale à la quantité de liquide de dialyse usagé (ou second liquide) refoulé par le dispositif de pompage selon l'invention et d'autre part que les débits de liquide de dialyse sont continus dans les canalisations 18 et 19, c'est à dire à l'entrée et à la sortie de l'hémodialyseur (courbes 5 et 6).

Toutes les courbes ont en abcisse le temps, découpé en phases successives d'aspiration et de refoulement, et on ordonnée le débit exprimé en Unité de débit. On peut par exemple, choisir une Unité de Débit égale à 500 ml/mn.

Les courbes de la figure 4 correspondent aux variations de débit dans les canalisations de liquide de dialyse frais alors que les courbes de la figure 5 correspondent aux variations de débit dans les canalisations de liquide de dialyse usagé correspondantes.

La courbe 1 de la figure 4 montre que l'alimentation du circuit à partir de la source de liquide de dialyse frais se fait avec un débit continu. Que l'on soit en phase d'aspiration ou de refoulement, on a toujours 1 Unité de débit dans la canalisation 20, en amont de l'embranchement avec la canalisation 23.

La courbe 2 est représentative de la circulation dans la canalisation 20 en aval de l'embranchement avec la canalisation 23. On voit que quand la vanne 28 est ouverte, c'est à dire en phase d'aspiration, le débit de liquide de dialyse est de 2 Unités, 1 Unité provenant de la chambre auxiliaire 17 par l'intermédiaire de la canalisation 23 et 1 Unité provenant de la source du liquide de dialyse, alors qu'il est nul en phase de refoulement, c'est à dire quand la vanne 28 est fermée.

Par contre, on voit sur la courbe 3, que dans la canalisation 18 en amont de l'embranchement avec la canalisation 24, le débit est nul en phase d'aspiration (vanne 26 fermée) et est égal à 2 Unités en phase de refoulement (vanne 26 ouverte); les 2 Unités provenant de la variation de volume de la chambre principale 12 et se répartissant ensuite en 1 Unité dans la canalisation 24 à destination de la chambre auxiliaire 15 et 1 Unité vers l'entrée de l'hémodialyseur.

La courbe 4 représente le débit dans la canalisation 24. En aspiration, le débit est de "+" 1 Unité, c'est à dire que le liquide de dialyse s'écoule de la chambre 15 vers la canalisation 18, alors qu'en phase de refoulement, il est de "−" 1 Unité, le signe négatif signifiant que le liquide s'écoule alors de la canalisation 18 vers la chambre auxiliaire 15.

La courbe 5 représente le débit de liquide de dialyse qui entre dans l'hémodialyseur. Il peut être considéré comme la somme des débits des courbes 3 et 4. On voit alors que le débit à l'entrée de l'hémodialyseur est continu et égal au débit fourni par la source de liquide de dialyse frais.

En se référant à la courbe 6 de la figure 5, on voit que le débit de liquide de dialyse usagé dans la canalisation 19 de sortie de l'hémodialyseur, en amont de l'embranchement de la canalisation 25 est continu et égal à 1 Unité. Ce débit peut être considéré comme la somme des débits des courbes 7 et 8.

La courbe 7 représente le débit de liquide de dialyse dans la canalisation 25. En phase d'aspiration, le débit est de "+" 1 Unité, c'est-à-dire que le liquide s'écoule de la canalisation 19 vers la chambre auxiliaire 16, alors qu'en phase de refoulement, le débit est de "−" 1 Unité, le liquide s'écoulant alors de la chambre auxiliaire 16 vers la chambre principale 13.

La courbe 8 représente le débit de liquide dans la canalisation 19 en aval de l'embranchement avec la canalisation 25. En phase d'aspiration, c'est à dire quand la vanne 27 est fermée, le débit est nul; en phase de refoulement, c'est à dire quand la vanne 27 est ouverte, le débit est alors égal à 2 Unités, 1 Unité provenant de la chambre auxiliaire 16 par l'intermédiaire de la canalisation 25 et 1 Unité provenant de l'hémodialyseur.

La courbe 9 représent le débit dans la canalisation 21 en amont de l'embranchement avec la canalisation 22. En phase d'aspiration, c'est-à-dire quand la vanne 29 est ouverte, le débit est égal à 2 Unités provenant de la variation de volume de la chambre principale 13. En phase de refoulement, c'est à dire quand la vanne 29 est fermée, le débit est nul.

La courbe 10 représente le débit dans la canalisation 21, en aval de l'embranchement avec la canalisation 22; c'est le débit de liquide de dialyse usagé qui est conduit à des moyens d'évacuation ou de régénération. C'est un débit continu égal à 1 Unité

La comparaison des courbes 1 et 10 montre que la quantité de liquide qui entre par la canalisation 20 est égale à la quantité de liquide qui sort par la canalisation 21.

D'autre part, les courbes 5 et 6, représentatives de la circulation du liquide de dialyse dans les canalisation 18 et 19, mettent en évidence le fait que le liquide de dialyse traverse l'hémodialyseur avec un débit continu.

La quantité de liquide de dialyse aspiré et refoulé durant chaque phase dépend de l'amplitude du mouvement du piston 9. La quantité totale de liquide de dialyse traversant le circuit sur l'ensemble de la séance de traitement dépend de la valeur du débit et donc de la fréquence des phases d'aspiration et de refoulement, c'est à dire de la vitesse à laquelle est animée la tige 30.

Sur la figure 3, on voit qu'une pompe 31, appelée pompe d'ultrafiltration, retire du circuit une quantité de liquide de dialyse égale à la quantité de liquide que l'on souhaite éliminer du sang du patient par ultrafiltration.

En effet, dans le cas où le circuit de liquide de dialyse est un circuit indéformable et fermé du point de vue des pressions, toute quantité de liquide retirée du circuit de liquide de dialyse provoque dans le circuit une dépression qui entraine, au niveau de l'hémodialyseur un gradient de pression de part et d'autre de la membrane. Ce gradient de pression provoque le passage à travers la membrane semi-perméable de l'hémodialyseur d'une quantité d'ultrafiltrat provenant du sang égale à la quantité de liquide retirée du circuit de liquide de dialyse.

Le liquide de dialyse peut être extrait à partir de la canalisation 19, en amont de l'embranchement avec la canalisation 25 ainsi que cela est représenté sur la figure 3, mais également à partir de la canalisation 18 en aval de l'embranchement avec la canalisation 24.

On peut, en outre, prévoir sur le circuit de liquide de dialyse, bien qu'ils ne soient pas représentés, des dispositifs de dégazage, des capteurs de pression ou de débit, ou tout autre accessoire non critique pour la présente invention.

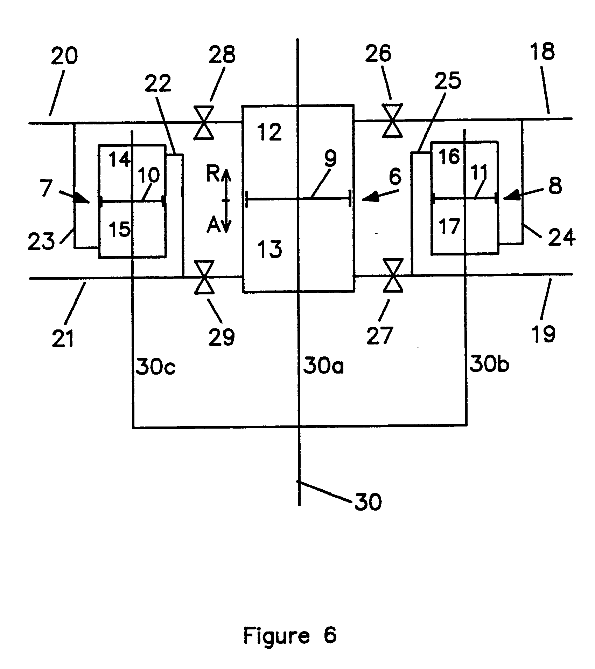

Selon le mode de réalisation représenté à la figure 6, les cylindres auxiliaires 7 et 8 ne sont plus disposés dans le même axe que le cylindre principal 6. L'entrainement des pistons ne se fait pas au moyen d'une tige unique, mais par exemple, au moyen de trois tiges 30a, 30b, 30c reliées à une tige 30 qui est animée d'un mouvement rectiligne alternatif par tout moyen connu à la portée de l'homme du métier.

Dans le cas où, comme représenté à la figure 6, les cylindres 6, 7 et 8 sont des cylindres de section circulaire, on choisit par exemple les pistons 10 et 11 avec une section égale à la moitié de la section du piston principal. Ainsi, lorsque les pistons 10 et 11 sont animés du même mouvement avec la même amplitude, que le piston principal 9, la quantité de liquide déplacé dans chacune des chambres principales est double de la quantité de liquide déplacé simultanément dans chacune des chambres auxiliaires.

La figure 6 illustre en outre le fait que l'on peut intervertir le rôle des chambres auxiliaires 15 et 17. En effet, selon ce mode de réalisation, la chambre 15, complémentaire de la chambre 14, est reliée par une canalisation 23 à la canalisation 20 d'aspiration du premier liquide, alors que la chambre auxiliaire 17, complémentaire de la chambre auxiliaire 16, est reliée par une canalisation 24 à la canalisation 18 de refoulement du premier liquide. De la même façon, il est possible d'intervertir le rôle des chambres 14 et 16.

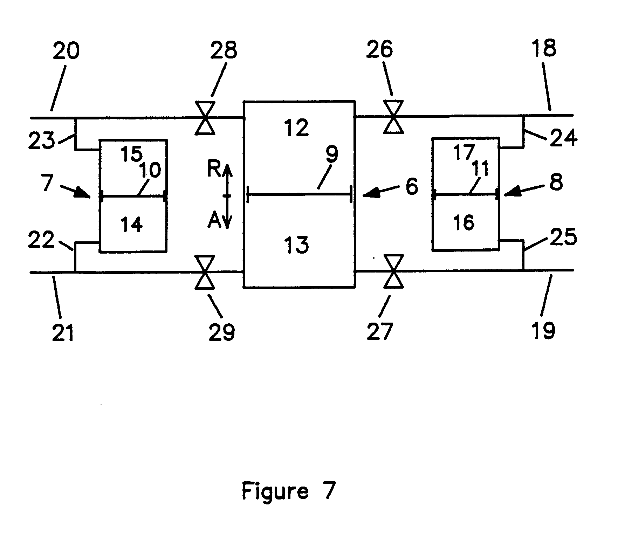

Le schéma de la figure 7 illustre une autre disposition relative des chambres auxiliaires (14, 15, 16, 17). Dans ce cas, les pistons auxiliaires 10 et 11, se déplacent en même temps et avec la même amplitude que le piston principal 9, mais en sens contraire. Ainsi, quand le piston principal 9 se déplace dans le sens de la flèche A, les pistons auxiliaires 10 et 11 se déplacent dans le sens de la flèche R et inversement. Ceci peut être réalise, par exemple, au moyen de systèmes mécaniques bielle-manivelle. Dans ce cas, la chambre auxiliaire 14 est reliée par une canalisation 22 à la canalisation 21 de refoulement du second liquide en aval du dispositif d'obturation 29. La chambre auxiliaire 15 qui lui est complémentaire, est reliée par une canalisation 23 à la canalisation 20 d'aspiration du premier liquide, en amont du dispositif d'obturation 28.

La chambre auxiliaire 16 est reliée par une canalisation 25 à la canalisation 19 d'aspiration du second liquide en amont du dispositif d'obturation 27. La chambre auxiliaire 17 qui lui est complémentaire est relié par une canalisation 24 à la canalisation 18 de refoulement du premier liquide.

Le fonctionnement du dispositif selon ce mode de réalisation est le suivant:

En phase d'aspiration, les vannes 26 et 27 sont fermées alors que les vannes 28 et 29 sont ouvertes. Le piston principal 9 se déplace dans le sens de la flèche A, alors que les pistons auxiliaires 10 et 11 se déplacent dans le sens de la flèche R.

L'augmentation de volume de la chambre principale 12 entraine une aspiration du premier liquide provenant pour moitié de la canalisation 20 d'aspiration du premier liquide et pour moitié de la chambre 15, par la canalisation 23. La diminution de volume de la chambre principale 13 entraine le refoulement du second liquide pour moitié vers la chambre 14 par l'intermédiaire de la canalisation 22, et pour moitié vers les moyens d'évacuation par la canalisation de refoulement 21. Simultanément, le mouvement du piston 11 refoule à partir de la chambre auxiliaire 17 le premier liquide vers la canalisation 18 de refoulement du premier liquide, par l'intermédiaire de la canalisation 24, et aspire dans la chambre auxiliaire 16, par l'intermédiaire de la canalisation 25 le second liquide provenant de la canalisation 19 d'aspiration du second liquide.

En phase de refoulement, les vannes 26 et 27 sont ouvertes alors que les vannes 28 et 29 sont fermées. Le piston 9 se déplace dans le sens de la flèche R alors que les pistons 10 et 11 se déplacent dans le sens de la flèche A.

Le premier liquide qui est chassé de la chambre principale 12 par le piston 9 est refoulé dans la chambre auxiliaire 17 par l'intermédiaire de la canalisation 24 et dans la canalisation 18 de refoulement du second liquide. L'augmentation de volume de la chambre principale 13 entraine une aspiration du second liquide qui provient pour moitié de la chambre auxiliaire 16 par l'intermédiaire de la canalisation 25 et pour moitié de la canalisation 19 d'aspiration du second liquide. Simultanément, le mouvement du piston 10 entraine le refoulement du second liquide à partir de la chambre auxiliaire 14 vers la canalisation 21 de refoulement par l'intermédiaire de la canalisation 22, ainsi que l'aspiration dans la chambre 15 du premier liquide provenant de la canalisation d'aspiration 20 par l'intermédiaire de la canalisation 23.

Un tel mode de réalisation permet, de la même façon que ceux des figures 3 et 6 de pomper des quantités égales d'un premier et d'un second liquides en maintenant dans les canalisations d'aspiration (19, 20) et de refoulement (18, 21) un débit continu.

De nombreuses variantes de réalisation sont à la portée du technicien sans sortir du cadre de la présente invention.

Ainsi l'entrainement des pistons peut être mécanique, ainsi que cela a été décrit, mais aussi magnétique, électromagnétique ou hydraulique.

Dans le cas d'un entrainement hydraulique, c'est-à-dire au moyen de pompes, par exemple une pompe sur la canalisation 20 d'aspiration du premier liquide en amont de l'embranchement avec la canalisation 23, et une pompe sur la canalisation d'aspiration du second liquide en amont de l'embranchement avec la canalisation 25, le dispositif principal 6 ne joue plus un rôle moteur pour le pompage. Il est également possible, bien que cela ne soit par préférentiel selon la présente invention, de remplacer les pistons par des membranes étanches ou diaphragmes.

Les vannes (26, 27, 28, 29) qui sont de façon préférentielle des vannes commandées, par exemple des électrovannes, peuvent être remplacées par tout dispositif d'obturation connu en soi, par exemple par des clapets anti-retour. Cependant dans le cas d'un entrainement hydraulique des pistons, on risquerait un passage en continu du premier liquide de la canalisation d'aspiration 20 vers la canalisation de refoulement 18 ainsi que du second liquide de la canalisation d'aspiration 19 vers la canalisation de refoulement 21.

On peut également remplacer l'ensemble des quatre vannes (26, 27, 28, 29) par tout moyen équivalent tel qu'un distributeur permettant de sélectionner les voies de passage du liquide de dialyse.

Dans les modes de réalisation représentés aux figures 3, 6 et 7, on a choisi pour les dispositifs 6, 7 et 8 une forme cylindrique de section circulaire, avec en outre une section droite du cylindre principal 6 double de la section droite de chacun des cylindres auxiliaires 7 et 8. Il est cependant possible, de choisir pour les cylindres auxiliaires une section droite non circulaire, ou de surface différente à condition de modifier en conséquence l'amplitude du mouvement des pistons auxiliaires, de façon à ce que la quantité de liquide déplacé lors de la variation de volume de chacune des chambres principales suite au déplacement du piston 9 soit double de la quantité de liquide déplacé simultanément lors de la variation de volume de chacune des chambres auxiliaires correspondantes, suite au déplacement des pistons auxiliaires 10 et 11.

Il est également possible, bien que cela ne soit pas préférentiel pour l'objet de la présente invention, que l'un ou plusieurs des dispositifs 6, 7, 8 soit constitué, non pas d'un cylindre divisé en deux chambres complémentaires grâce à un piston, mais d'un système à balancier conçu de telle façon que les deux chambres associées soient complémentaires l'une de l'autre.

Parmi les applications possibles du procédé et du dispositif selon l'invention, on a décrit, en se référant à la figure 3, le pompage de quantités égales de liquide de dialyse frais et de liquide de dialyse usagé.

On peut également utiliser l'objet de la présente invention dans le cadre de l'hémofiltration avec réinjection. En effet, dans cette technique, la quantité de liquide retirée du sang par ultrafiltration est remplacée, à l'ajustement près du poid du patient, par une égale de liquide de substitution qui est injectée dans le sang. Le premier liquide pompé est alors le liquide ultrafiltré et le second liquide est le liquide de substitution.

**Revendications**

1. Procédé de pompage pour rein artificiel d'un premier et d'un second liquide en quantités égales et avec un débit continu selon lequel on aspire et on refoule simultanément, en quantités égales, un premier et un second liquide grâce à un dispositif principal de pompage (6) à deux chambres principales complémentaires (12, 13) la première chambre (12) étant destinée au premier liquide et communiquant avec une canalisation d'aspiration (20) par un premier dispositif d'obturation (28) et avec une canalisation de refoulement (18) par un second dispositif d'obturation (26), la seconde chambre (13) étant destinée au second liquide et communiquant avec une canalisation d'aspiration (19) par un troisième dispositif d'obturation (27) et avec une canalisation de refoulement (21) par un quatrième dispositif d'obturation (29), caractérisé en ce que:

—on régule le débit du premier liquide grâce à une capacité de régulation comprenant deux chambres auxiliaires (15, 17) dont les volumes varient simultanément et en sens opposé du volume de la chambre principale (12) correspondante, la quantité de liquide déplacé lors de la variation de volume de la chambre principale (12) étant double de la quantité de liquide déplacé lors de la variation de volume de chacune des chambres auxiliaires, l'une des chambres auxiliaires étant reliée à la canalisation (20) d'aspiration du premier liquide alors que l'autre chambre auxiliaire est reliée à la canalisation (18) de refoulement dudit premier liquide,

—on régule le débit du second liquide grâce à une capacité de régulation comprenant deux chambres auxiliaires (14, 16) dont les volumes varient simultanément et en sens opposé du volume de la principale (13) correspondante, la quantité de liquide déplacé lors de la variation de volume de la chambre principale (13) étant double de la quantité de liquide déplacé lors de la variation de volume de chacune des chambres auxiliaires correspondantes, l'une des chambres auxiliaires étant reliée à la canalisation (19) d'aspiration du second liquide, alors que l'autre auxiliaire est relié à la canalisation (21) de refoulement dudit second liquide.

2. Procédé selon la revendication 1, caractérisé en ce que le premier liquide est du liquide de dialyse frais et le second liquide est du liquide de dialyse usagé ou inversement.

3. Procédé selon la revendication 1, caractérisé en ce que le premier liquide correspond au liquide ultrafiltré et le second liquide est du liquide de substitution ou inversement.

4. Dispositif de pompage pour rein artificiel d'un premier et d'un second liquide en quantités égales et avec un débit continu comprenant un dispositif principal de pompage (6) à deux chambres principales complémentaires (12, 13), la chambre (12) destinée au premier liquide communiquant par un dispositif d'obturation (28) avec une canalisation (20) d'aspiration du premier liquide et par un dispositif d'obturation (26) avec une canalisation (18) de refoulement du premier liquide, la chambre (13) destinée au second liquide communiquant par un dispositif d'obturation (27) avec une canalisation (19) d'aspiration du second liquide et par un dispositif d'obturation (29) avec une canalisation (21) de refoulement du second liquide, caractérisé en ce qu'il comporte en outre:

—une capacité de régulation du premier liquide comprenant deux chambres auxiliaires (15, 17) dont les volumes varient simultanément et en sens opposé du volume de la chambre principale correspondante (12), la quantité de liquide déplace lors de la variations de volume de la chambre principale (12) étant double de la quantité de liquide déplacé lors de la variation de volume de chacune des chambres auxiliaires, l'une des chambres auxiliaires étant reliée à la canalisation (20) d'aspiration du premier liquide en amont d'un premier dispositif d'obturation (28) alors que l'autre chambre auxiliaire est reliée à la canalisation (18) de refoulement du premier liquide en aval d'un second dispositif d'obturation (26),

—une capacité de régulation du second liquide comprenant deux chambres auxiliaires (14, 16) dont les volumes varient simultanément et en sens opposé du volume de la chambre principale correspondante (13), la quantité de liquide déplacé lors de la variation de volume de la chambre principale (13) étant double de la quantité de liquide déplacé lors de la variation de volume de chacune des chambres auxiliaires, l'une des chambres auxiliaires étant reliée à la canalisation (19) d'aspiration du second liquide en amont d'un troisième dispositif d'obturation (27) alors que l'autre chambre auxiliaire est reliée à la canalisation (21) de refoulement du second liquide en aval d'un quatrième dispositif d'obturation (29),

—des moyens d'entrainement du dispositif principal de pompage (6).

5. Dispositif selon la revendication 4, caractérisé en ce que des pistons (9, 10, 11) entraînent une variation de volume des chambres (12, 13, 14, 15, 16, 17).

6. Dispositif selon la revendication 4, caractérisé en ce que des diaphragmes (9, 10, 11) délimitent les chambres (12, 13, 14, 15, 16, 17) de façon étanche.

7. Dispositif selon la revendication 4 ou 5, caractérisé en ce que le dispositif principal de pompage (6) comprend un cylindre principal de section droite circulaire, divisé en deux chambres principales (12, 13) par un piston (9) mobile.

8. Dispositif selon la revendication 5 ou 7, caractérisé en ce que les pistons (9, 10, 11) sont coaxiaux.

9. Dispositif selon l'une quelconque des revendications 4 à 8 caractérisé en ce que le dispositif principal de pompage (6) est moteur.

10. Dispositif selon l'une quelconque des revendications 4 à 9, caractérisé en ce que les moyens d'entrainement du dispositif principal de pompage sont du type électromagnétique.

11. Dispositif selon l'une quelconque des revendications 4 à 9, caractérisé en ce que les moyens d'entrainement du dispositif principal de pompage sont du type mécanique.

12. Dispositif selon l'une quelconque des revendications 4 à 11, caractérisé en ce que les capacités de régulation comprennent chacune deux chambres auxiliaires cylindriques de section droite circulaire.

13. Dispositif selon l'une quelconque des revendications 4 à 12, caractérisé en ce que la section droite des chambres principales (12, 13) est double de la section droite des chambres auxiliaires (14, 15, 16, 17).

14. Dispositif selon l'une quelconque des revendications 4 à 13, caractérisé en ce que les dispositif d'obturation (26, 27, 28, 29) sont des vannes commandées.

15. Dispositif selon l'une quelconque des revendications 4 à 13 caractérisé en ce que les dispositifs d'obturation (26, 27, 28, 29) sont regroupés à l'intérieur d'un distributeur.

**Patentansprüche**

1. Verfahren für eine künstliche Niere zum Pumpen einer ersten und einer zweiten Flüssigkeit in gleichen Mengen und mit einem kontinuierlichen Durchsatz, bei dem gleichzeitig gleiche Mengen einer ersten und einer zweiten Flüssigkeit angesaugt und abgegeben werden mit Hilfe einer Hauptpumpvorrichtung (6) mit zwei komplementären Hauptkammern (12, 13), wobei die erste Kammer (12) für die erste Flüssigkeit bestimmt ist und in Verbindung steht mit einer Ansaugleitung (20) durch eine erste Absperreinrichtung (28) und mit einer Abgabeleitung (18) durch eine zweite Absperreinrichtung (26), und die zweite Kammer (13) für die zweite Flüssigkeit bestimmt ist und mit einer Ansaugleitung (19) durch eine dritte Absperreinrichtung (27) und mit einer Abgabeleitung (21) durch eine vierte Absperreinrichtung (29) in Verbindung steht, dadurch gekennzeichnet, daß

—der Durchsatz der ersten Flüssigkeit gesteuert wird mit Hilfe einer Regelkapazität mit zwei Hilfskammern (15, 17), deren Volumen sich gleichzeitig und entgegengesetzt dem Volumen der entsprechenden Hauptkammer (12) verändern, wobei die Menge der während der Volumenänderung der Hauptkammer (12) verdrängten Flüssigkeit doppelt so groß ist wie die Menge der Flüssigkeit, die während der Volumenänderung jeder der Hilfskammern verdrängt wird, wobei eine der Hilfskammern verbunden ist mit der Ansaugleitung (20) für die erste Flüssigkeit, während die andere Hilfskammer mit der Abgabeleitung (18) für die erste Flüssigkeit verbunden ist,

—der Durchsatz der zweiten Flüssigkeit gesteuert wird mit Hilfe einer Regelkapazität mit zwei Hilfskammern (14, 16), deren Volumen sich gleichzeitig und entgegengesetzt dem Volumen der entsprechenden Hauptkammer (13) verändern, wobei die Menge der während der Volumenänderung der Hauptkammer (13) verdrängten Flüssigkeit doppelt so groß ist wie die Menge der Flüssigkeit, die während der Volumenänderung jeder der entsprechenden Hilfskammern verdrängt wird, wobei eine der Hilfskammern verbunden ist mit der Ansaugleitung (19) für die zweite Flüssigkeit, während die andere Hilfskammer mit der Abgabeleitung (21) für die zweite Flüssigkeit verbunden ist.

2. Verfahren nach Anspruch 1, dadurch gekennzeichnet, daß die erste Flüssigkeit frische und die zweite Flüssigkeit gebrauchte Dialyseflüssigkeit ist oder umgekehrt.

3. Verfahren nach Anspruch 1, dadurch gekennzeichnet, daß die erste Flüssigkeit ultrafiltrierter Flüssigkeit entspricht und die zweite Flüssigkeit einer Austauschflüssigkeit ist oder umgekehrt.

4. Vorrichtung für eine künstliche Niere zum Pumpen einer ersten und einer zweiten Flüssigkeit in gleichen Mengen und mit einem kontinuierlichen Durchsatz mit einer Hauptpumpvorrichtung (6) mit zwei komplementären Hauptkammern (12, 13), wobei die Kammer (12) für die erste Flüssigkeit bestimmt ist und durch eine Absperreinrichtung (28) mit einer Ansaugleitung (20) für die erste Flüssigkeit und durch eine Absperreinrichtung (26) mit einer Abgabeleitung (18) für die erste Flüssigkeit in Verbindung steht, und wobei die Kammer (13) für die zweite Flüssigkeit bestimmt ist und durch eine Absperreinrichtung (27) mit einer Ansaugleitung (19) für die zweite Flüssigkeit und durch eine Absperreinrichtung (29) mit einer Abgabeleitung (21) für die zweite Flüssigkeit in Verbindung steht, dadurch gekennzeichnet, daß sie außerdem umfaßt

—eine Regelkapazität für die erste Flüssigkeit mit zwei Hilfskammern (15, 17), deren Volumen sich gleichzeitig und entgegengesetzt dem Volumen der entsprechenden Hauptkammer (12) verändern, wobei die Menge der während der Volumenveränderung der Hauptkammer (12) verdrängten Flüssigkeit doppelt so groß ist wie die Menge der Flüssigkeit, die während der Volumenänderung jeder der Hilfskammern verdrängt wird, und wobei eine der Hilfskammern verbunden ist mit der Ansaugleitung (20) für die erste Flüssigkeit stromaufwärts einer ersten Absperreinrichtung (28) während die andere Hilfskammer mit der Abgabeleitung (18) für die erste Flüssigkeit stromabwärts einer zweiten Absperreinrichtung (26) verbunden ist,

—eine Regelkapazität für die zweite Flüssigkeit mit zwei Hilfskammern (14, 16), deren Volumen sich gleichzeitig und entgegengesetzt dem Volumen der entsprechenden Hauptkammer (13) verändern, wobei die Menge der während der Volumenänderung der Hauptkammer (13) verdrängten Flüssigkeit doppelt so groß ist wie die Menge der Flüssigkeit, die während der Volumenänderung jeder der Hilfskammern verdrängt wird, und wobei eine der Hilfskammern verbunden ist mit der Ansaugleitung (19) für die zweite Flüssigkeit stromaufwärts einer dritten Absperreinrichtung (27), während die andere Hilfskammer mit der Abgabeleitung (21) für die zweite Flüssigkeit stromabwärts einer vierten Absperreinrichtung (29) verbunden ist, und

—Antriebseinrichtungen für die Hauptpumpvorrichtung (6).

5. Vorrichtung nach Anspruch 4, dadurch gekennzeichnet, daß Kolben (9, 10, 11) eine Volumenveränderung der Kammern (12, 13, 14, 15, 16, 17) herbeiführen.

6. Vorrichtung nach Anspruch 4, dadurch gekennzeichnet, daß Membranen (9, 10, 11) die Kammern (12, 13, 14, 15, 16, 17) dicht abschließen.

7. Vorrichtung nach Anspruch 4 oder 5, dadurch gekennzeichnet, daß die Hauptpumpvorrichtung (6) einen Hauptzylinder mit rundem Querschnitt aufweist, der durch einen bewegbaren Kolben in zwei Hauptkammern (12, 13) geteilt ist.

8. Vorrichtung nach Anspruch 5 oder 7, dadurch gekennzeichnet, daß die Kolben (9, 10, 11) koaxial sind.

9. Vorrichtung nach einem der Ansprüche 4 bis 8, dadurch gekennzeichnet, daß die Hauptpumpvorrichtung (6) ein Motor ist.

10. Vorrichtung nach einem der Ansprüche 4 bis 9, dadurch gekennzeichnet, daß die Antriebseinrichtung für die Hauptpumpvorrichtung elektromagnetisch ist.

11. Vorrichtung nach einem der Ansprüche 4 bis 9, dadurch gekennzeichnet, daß die Antriebseinrichtung für die Hauptpumpvorrichtung mechanisch ist.

12. Vorrichtung nach einem der Ansprüche 4 bis 11, dadurch gekennzeichnet, daß die Regelkapazitäten jeweils zwei zylindrische Hilfskammern mit rundem Querschnitt aufweisen.

13. Vorrichtung nach einem der Ansprüche 4 bis 12, dadurch gekennzeichnet, daß der Querschnitt der Hauptkammern (12, 13) doppelt so groß ist wie der Querschnitt der Hilfskammern (14, 15, 16, 17).

14. Vorrichtung nach einem der Ansprüche 4 bis 13, dadurch gekennzeichnet, daß die Absperreinrichtungen (26, 27, 28, 29) Steuerventile sind.

15. Vorrichtung nach einem der Ansprüche 4 bis 13, dadurch gekennzeichnet, daß die Absperreinrichtungen (26, 27, 28, 29) im Innern eines Verteilers gruppiert sind.

**Claims**

1. A method for pumping a first and a second liquid for an artificial kidney in equal quantities and with a continuous flow, according to which one simultaneously draws in and delivers a first and a second liquid in equal quantities by means of a main pumping device (6) with two main complementary chambers (12, 13), the first chamber (12) being intended for a first liquid and communicating with an aspiration line (20) via a first obturating device (28) and with a delivery line (18) via a second obturating device (26), the second chamber (13) being intended for the second liquid and communicating with an aspiration line (19) via a third obturating device (27) and with a delivery line (21) via a fourth obturating device (29), characterized in that:

—one regulates the flow of the first liquid by means of a regulating facility comprising two auxiliary chambers (15, 17) whose volumes vary simultaneously and inversely in relation to the volume of the corresponding main chamber (12), the quantity of the liquid displaced during the variation in volume of the main chamber (12) being twice the quantity of the liquid displaced during the variation in volume of each one of the auxiliary chambers, one of the auxiliary chambers being connected to the aspiration line (20) for the first liquid, whilst the other auxiliary chamber is connected to the delivery line (18) for the said first liquid,

—one regulates the flow of the second liquid by means of a regulating facility comprising two auxiliary chambers (14, 16) whose volumes vary simultaneously and inversely in relation to the volume of the corresponding main chamber (13), the quantity of the liquid displaced during the variation in volume of the main chamber (13) bein twice the quantity of the liquid displaced during the variation in volume of each one of the corresponding auxiliary chambers, one of the auxiliary chambers being connected to the aspiration line (19) for the second liquid, whilst the other auxiliary chamber is connected to the delivery line (21) for the said second liquid.

2. A method according to claim 1, characterized in that the first liquid is fresh dialysis liquid and the second liquid is used dialysis liquid or vice versa.

3. A method according to claim 1, characterized in that the first liquid corresponds to the ultrafiltrated liquid and the second liquid is the substitution liquid or vice versa.

4. A device for an artificial kidney for pumping a first and a second liquid in equal quantities and with a constant flow comprising a main pumping device (6) with two main complementary chambers (12, 13), the chamber (12) intended for the first liquid communicating via an obturating device (28) with an aspiration line (20) for the first liquid, and via an obturating device (26) with a delivery line (18) for the first liquid, the chamber (13) intended for the second liquid communicating via an obturating device (27) with an aspiration line (19) for the second liquid and via an obturating device (29) with a delivery line (21) for the second liquid, characterized in that it comprises moreover:

—a regulating facility for the first liquid comprising two auxiliary chambers (15, 17) whose volumes vary simultaneously and inversely in relation to the volume of the corresponding main chamber (12), the quantity of liquid displaced during the variation in volume of the main chamber (12) being twice the quantity of the liquid displaced during the variation in volume of each one of the auxiliary chambers, one of the auxiliary chambers being connected to the aspiration line (20) for the first liquid upstream from a first obturating device (28), whilst the other auxiliary chamber is connected to the delivery line (18) for the first liquid downstream from a second obturating device (26),

—a regulating facility for the second liquid comprising two auxiliary chambers (14, 16) whose volumes vary simultaneously and inversely in relation to the volume of the corresponding main chamber (13), the quantity of liquid displaced during the variation in volume of the main chamber (13) being twice the quantity of the liquid displaced during the variation in volume of each one of the auxiliary chambers, one of the auxiliary chambers being connected to the aspiration line (19) for the second liquid upstream from a third obturating device (27) whilst the other auxiliary chamber is connected to the delivery line (21) for the second liquid downstream from a fourth obturating device (29),

—means for driving the main pumping device (6).

5. A device according to claim 4, characterized in that pistons (9, 10, 11) produce a variation in volume of the chambers (12, 13, 14, 15, 16, 17).

6. A device according to claim 4, characterized in that diaphragms (9, 10, 11) delimit the chambers (12, 13, 14, 15, 16, 17) in a leakproof manner.

7. A device according to claim 4 or 5, characterized in that the main pumping device (6) comprises a main cylinder with a circular cross section divided into two main chambers (12, 13) by a movable piston (9).

8. A device according to claim 5 or 7, characterized in that the pistons (9, 10, 11) are coaxial.

9. A device according to any one of claims 4 to 8, characterized in that the main pumping device (6) is the motor.

10. A device according to any one of claims 4 to 9, characterized in that the means for driving the main pumping device are of an electromagnetic type.

11. A device according to any one of claims 4 to 9, characterized in that the means for driving the main pumping device are of a mechanical type.

12. A device according to any one of claims 4 to 11, characterized in that the regulating facilities each comprise two cylindrical auxiliary chambers with a circular cross section.

13. A device according to any one of claims 4 to 12, characterized in that the cross section of the main chambers (12, 13) is twice the cross section of the auxiliary chambers (14, 15, 16, 17).

14. A device according to any one of claims 4 to 13, characterized in that the obturating devices (26, 27, 28, 29) are controlled valves.

15. A device according to any one of claims 4 to 13, characterized in that the obturating devices (26, 27, 28, 29) are grouped together within a distributor.

Figure 1

Figure 2

Figure 3

Figure  4

Figure 5

Figure 6

Figure 7